# EUROPÄISCHE PATENTSCHRIFT

(11) **EP 3 600 231 B1**
(45) Veröffentlichungstag und Bekanntmachung des Hinweises auf die Patenterteilung: **16.07.2025**
(21) Anmeldenummer: 18711509.2
(22) Anmeldetag: 09.03.2018
(51) Int. Cl.: A61K 8/34, A61Q 5/06, A61K 8/73, A61K 8/81, A61K 8/02, A61K 8/04

(54) **SPRÜHBARES KOSMETISCHES MITTEL I**
SPRAYABLE COSMETIC AGENT I
AGENT COSMÉTIQUE PULVÉRISABLE I

(30) Priorität: 31.03.2017 DE 102017205558; 21.11.2017 DE 102017220773
(43) Veröffentlichungstag der Anmeldung: 05.02.2020
(73) Patentinhaber: Henkel AG & Co. KGaA, 40589 Düsseldorf (DE)
(72) Erfinder: METTEN, Diane, 22393 Hamburg (DE); SCHEFFLER, Rene, 25479 Ellerau (DE); LANGE, Julia Bibiane, 24576 Bad Bramstedt (DE); MARTINEZ, Cyrielle, 22763 Hamburg (DE); KNAPPE, Thorsten, 22869 Schenefeld (DE); BAYERSDÖRFER, Rolf, 22589 Hamburg (DE)
(86) Internationale Anmeldenummer: PCT/EP2018/055893
(87) Internationale Veröffentlichungsnummer: WO 2018/177722

(56) Entgegenhaltungen:
- WO-A1-2017/178855
- DE-A1- 19 640 099
- JP-A- H11 158 034
- US-A1- 2005 042 192
- ANONYMOUS: "D.S.A 7 RED", 4 October 2016 (2016-10-04), XP055469654, Retrieved from the Internet <URL:http://www.agrana.com/fileadmin/inhalte/agrana_group/2017/images/Starch/Folder/Kosmetik/PDB_D.S.A.7_9017_en.pdf> [retrieved on 20180423]
- ANONYMOUS: "Dry Shampoo black & conditioner with D.S.A.7 black 20%", 5 December 2016 (2016-12-05), XP055469665, Retrieved from the Internet <URL:https://services.agrana.com/fileadmin/inhalte/austria/products/Kosmetik_Formulierungen/2018_Kosmetikdatein_Download/2018_Formulierungen/AGRANA_-_Dry_Shampoo_black_and_conditioner_with_D.S.A._7_black_20.pdf> [retrieved on 20180423]

## Beschreibung

Die vorliegende Anmeldung betrifft sprühbare kosmetische Zusammensetzung zur Behandlung keratinischer Fasern, welche neben einem Lösungsmittel und einem Treibmittel weiterhin Fasern enthalten. Diese Zusammensetzungen eignen sich für vielfältige kosmetische Zwecke, insbesondere jedoch zur temporären Verformung der keratinischen Fasern. Weiterhin betrifft die Anmeldung die kosmetische Verwendung dieser Zusammensetzungen und haarkosmetische Verfahren unter Einsatz dieser Zusammensetzungen.

Stylingmittel zur Verformung keratinhaltiger Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Zusammensetzungen zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufrieden stellender Genauigkeit dosieren. So lassen sich etwa Haargele, Haarcremes und Haarwachse nur noch schwierig verteilen, sobald sie einmal auf das Haar aufgebracht sind.

Haarsprays lassen sich gleichmäßiger auf das Haar verteilen, allerdings besteht auch bei den Applikationseigenschaften dieser sprühbaren Haarkosmetika deutliches Verbesserungspotential.

Die DE 196 40 099 A1 offenbart kosmetische Zusammensetzungen zur Behandlung keratinischer Fasern enthaltend Fasern (Anspruch 1, 8). Die Beispiele 4,11,12,17-20 solche Zusammensetzungen, die Seiden und/oder Polyamidfasern, Ethanol, mindestens ein Polymer (beispielsweise Dimethicon, Hydroxypropylmethylcellulose, VP/VA Copolymer etc.) sowie Treibmittel (Propan/Butan bzw. Dimethylether) enthalten.

Die Aufgabe der vorliegenden Erfindung war es nun, sprühbare Haarkosmetika bereitzustellen, welche sich gegenüber herkömmlichen Zubereitungen durch verbesserte Applikationseigenschaften, insbesondere ein verbessertes Sprühbild auszeichnen. Es hat sich gezeigt, dass diese Aufgabe durch den Zusatz von Fasern zu den sprühbaren kosmetischen Zubereitungen gelöst werden kann.

Durch die vorliegende Erfindung wird bereitgestellt:
Eine kosmetische Zusammensetzung zur kosmetischen Behandlung keratinischer Fasern, umfassend:
(a) Fasern, wobei die Fasern ausgewählt sind aus Baumwollfasern, Kapokfasern, Akonfasern, Pappelflaumfasern, Bambusfasern, Fasernessel, Hanffaser, Jutefaser, Flachsfaser, Sisalfaser, Kokosfaser und Fasern von Wolle, Alpaka, Kamelhaar, Amgora, Kaschmir, Mohair, Yakhaar, Ziegenhaar, Rinderhaar und Rosshaar, bevorzugt aus Cellulosefasern.
(b) Ethanol, wobei der Gewichtsanteil des Ethanols am Gesamtgewicht der kosmetischen Zusammensetzung 20 bis 50 Gew.-%, vorzugsweise 20 bis 45 Gew.-% und insbesondere 25 bis 40 Gew.-% beträgt,
(c) mindestens ein Polymer,
(d) Treibmittel,
(e) 0 bis 10 Gew.-% Farbstoff(e),
(f) 0 bis 10 Gew.-% Pigment(e),
(g) 1 ,0 bis 10 Gew.-%, mindestens einer Stärkeverbindung

enthält, mit der Maßgabe, dass
die Summe der Mengen der Inhaltsstoffe (e) und (f) 0,0001 bis 10 Gew.-%, bezogen auf das Gesamtgewicht der kosmetischen Zusammensetzung, beträgt und
wobei der Inhaltsstoff (e) und/oder (f) in Form eines Stärke-Farbstoff-Compounds und/oder eines Stärke-Pigment-Compounds, vorzugsweise in Form von gefärbter und/oder pigmentierter Reisstärke, enthalten ist.

2. Kosmetische Zusammensetzung nach Punkt 1, wobei der Gewichtsanteil der Fasern am Gesamtgewicht der kosmetischen Zusammensetzung 0, 1 bis 5,0, bevorzugt 0,1 bis 2,0 Gew.-%, besonders bevorzugt 0, 1 bis 1 ,0 Gew.-% und insbesondere 0, 1 bis 0,5 Gew.-% beträgt.

3. Kosmetische Zusammensetzung nach einem der vorherigen Punkte, wobei die Fasern ausgewählt sind aus der Gruppe der pflanzlichen Fasern, insbesondere der Cellulosefasern, vorzugsweise der Baumwollfasern, Flachsfasern und Kapopkfasern.

4. Kosmetische Zusammensetzung nach einem der vorherigen Punkte, wobei die Fasern eine Länge von 1 ,0 bis 200 µ 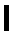 η, vorzugsweise von 2,0 bis 150 µ 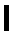 η und insbesondere von 5,0 bis 100 µ 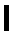 η aufweisen.

5. Kosmetische Zusammensetzung nach einem der vorherigen Punkte, wobei der Gewichtsanteil des mindestens einen Polymers am Gesamtgewicht der kosmetischen Zusammensetzung 0,5 bis 8,0 Gew.-%, vorzugsweise 1 ,0 bis 6,0 Gew.-% und insbesondere 2,0 bis 4,0 Gew.-% beträgt.

6. Kosmetische Zusammensetzung nach einem der vorherigen Punkte, wobei der Gewichtsanteil des Treibmittels am Gesamtgewicht des kosmetischen Zusammensetzung 30 bis 70 Gew.-%, vorzugweise 35 bis 65 Gew.-% und insbesondere 40 bis 60 Gew.-% beträgt.

7. Kosmetische Zusammensetzung nach einem der Punkte 2 bis 6, wobei sie - bezogen auf ihr Gesamtgewicht - 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.- % Farbstoff(e) enthält.

8. Kosmetische Zusammensetzung nach einem der Punkte 2 bis 7, wobei sie - bezogen auf ihr Gesamtgewicht - 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.- % Pigment(e) enthält.

9. Kosmetische Zusammensetzung nach einem der vorherigen Punkte 2 bis 8, wobei sie mindestens ein Pigment aus der Gruppe CI 12490, CI 14700, CI 14720, CI15510, CI15985, CI45380, CI47005, CI60730, CI61565, CI73360, CI74160, CI77007, CI77019, CI77288, CI77289, CI77491 , CI77492 enthält, wobei die Gesamtmenge an Pigment(en) aus dieser Gruppe 0,0001 bis 10 Gew.- %, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf das Gesamtgewicht der kosmetischen Zusammensetzung, beträgt.

10.Kosmetische Zusammensetzung nach einem der vorherigen Punkte, wobei sie als weiteren Bestandteil 2,0 bis 6,0 Gew.-% und insbesondere 3,0 bis 5,0 Gew.- %, jeweils bezogen auf das Gesamtgewicht der kosmetischen Zusammensetzung, mindestens einer Stärkeverbindung enthält.

11. Kosmetische Zusammensetzung nach einem der vorherigen Punkte 2 bis 10, wobei sie die Inhaltsstoffe (a) und (e) und/oder (a) und (f) in Form eines Faser-Farbstoff-Compounds oder eines Faser-Pigment-Compounds, vorzugsweise in Form von gefärbten und/oder pigmentierten Fasern, enthält.

12.Kosmetisches Mittel, umfassend
a) eine kosmetische Zusammensetzung nach einem der Punkte 1 bis 11
b) einen Aerosolabgabebehälter.

Die kosmetische Zusammensetzung enthält als ihren ersten wesentlichen Bestandteil Fasern, wobei die Fasern ausgewählt sind aus Baumwollfasern, Kapokfasern, Akonfasern, Pappelflaumfasern, Bambusfasern, Fasernessel, Hanffaser, Jutefaser, Flachsfaser, Sisalfaser, Kokosfaser und Fasern von Wolle, Alpaka, Kamelhaar, Amgora, Kaschmir, Mohair, Yakhaar, Ziegenhaar, Rinderhaar und Rosshaar, bevorzugt aus Cellulosefasern.

Der Zusatz dieser Fasern beeinflusst in nicht zu erwartender Weise die Applikationseigenschaften des sprühbaren Haarkosmetikums. Das Sprühbild und der Auftrag des versprühten Kosmetikums werden gleichmäßiger. In einer standardisierten Versuchsanordnung bewirkt der Zusatz der Fasern bei gleichem Druck in der Sprühvorrichtung und gleichem Abstand zu der zu besprühenden Fläche zudem eine überraschende Vergrößerung der erzielten Sprühfläche.

Als Fasern werden gestreckte, flexible Gebilde aus Fasermaterial mit einem Verhältnis von Länge zu Durchmesser oberhalb 3:1, vorzugsweise oberhalb 5:1 und insbesondere oberhalb 10:1. Fasern können in Längsrichtung keine Druck-, sondern nur Zugkräfte aufnehmen.

Die Länge bevorzugt eingesetzter Fasern liegt im Bereich von 1,0 bis 200 µm, vorzugsweise von 2,0 bis 150 µm und insbesondere von 5,0 bis 100 µm. Selbstverständlich sind aber auch Fasern größerer Faserlänge, beispielsweise Fasern mit einer Länge oberhalb 200µm oder oberhalb 300µm einsetzbar.

Pflanzenfasern kommen bei Pflanzen als Leitbündel im Stängel, im Stamm, in der Rinde oder als Samen-Fortsätze vor. Pflanzenfasern bestehen in der Regel zum überwiegenden Teil aus Cellulose. Entsprechende Fasern werden unter der Bezeichnung Cellulosefasern zusammengefasst.

Einer verbreiteten Unterteilung gemäß wird bei den Pflanzenfasern zwischen Samenfasern, Bastfasern, Blattfasern und Fruchtfasern unterschieden. Zur Gruppe der Samenfasern zählen beispielsweise die Baumwollfasern, Kapok, Akon oder Pappelflaum. Die Gruppe der Bastfasern schließt u.a. Bambusfaser, Fasernessel, Hanffaser, Jute und Flachsfaser mit ein. Sisal ist dem gegenüber den Blattfasern zuzurechnen, während die Kokosfasern zur Gruppe der Fruchtfasern gehören.

Als für die technische und kosmetische Wirkung besonders vorteilhaft haben sich Gewichtsanteile der Fasern am Gesamtgewicht der kosmetischen Zusammensetzung von 0,1 bis 5,0, bevorzugt 0,1 bis 2,0 Gew.-%, besonders bevorzugt 0,1 bis 1,0 Gew.-% und insbesondere 0,1 bis 0,5 Gew.- % erwiesen.

Als ihren zweiten wesentlichen Bestandteil enthalten die kosmetischen Zusammensetzungen Ethanol. Der Gewichtsanteil des Ethanols am Gesamtgewicht der kosmetischen Zusammensetzung beträgt vorzugsweise 45 bis 70 Gew.-%, bevorzugt 40 bis 65 Gew.-% und insbesondere 50 bis 60 Gew.-%. Die Fasern sind im Ethanol suspendiert.

Der dritte wesentliche Bestandteil der kosmetischen Zusammensetzung ist das mindestens eine Polymer. Für die Applizierbarkeit und kosmetische Wirkung der Zusammensetzungen hat es sich als vorteilhaft erwiesen, den Gewichtsanteil des Polymers am Gesamtgewicht der Zusammensetzung auf 0,5 bis 8,0 Gew.-%, vorzugsweise 1,0 bis 6,0 Gew.-% und insbesondere 2,0 bis 4,0 Gew.-% zu begrenzen.

Polymere werden in den kosmetischen Zusammensetzungen beispielsweise aufgrund ihrer festigenden und/oder filmbildenden Eigenschaften eingesetzt. Aufgrund ihrer kosmetischen Wirkung ist der Einsatz filmbildender Polymere besonders bevorzugt.

Als Polymere eignen sich permanent als auch temporär kationische, anionische, nichtionische oder amphotere Polymere. Die filmbildenden Polymere können synthetischen oder natürlichen Ursprungs sein.

Beispiele für gebräuchliche Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Mit besonderem Vorzug ist das Polymer ausgewählt aus der Gruppe der nichtionischen Polymere. Geeignete nichtionische Polymere sind beispielsweise:
- Polyvinylpyrrolidone, wie sie beispielsweise unter der Bezeichnung Luviskol^{®} (BASF) vertrieben werden.
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.

Erfindungsgemäß bevorzugt sind insbesondere die Polyvinylpyrrolidone und die Vinylpyrrolidon/Vinylacetat-Copolymere

Eine zweite Gruppe besonders bevorzugter Polymere bilden die amphoteren Polymere. Ganz besonders bevorzugt ist der Einsatz eines Copolymers von i) N-tert-Octylacrylamid, ii) Acrylsäure, iii) tert.-Butylaminoethylmethacrylat sowie gegebenenfalls weiteren Monomeren

Bevorzugte amphotere Copolymere bestehen bevorzugt zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylat. Entsprechende Copolymere können auch unter ausschließlicher Nutzung der Monomere N-tert-Octylacrylamid, Acrylsäure und tert.-Butylaminoethylmethacrylat erhalten werden.

Bevorzugt sind weiterhin Copolymere aus den Monomeren N-tert-Octylacrylamid, Acrylsäure, tert.-Butylaminoethylmethacrylat, Methylmethacrylat und Hydroxypropylmethacrylat. Besonders bevorzugt ist es insbesondere, wenn das Copolymer zu mindestens 90 Gew.-%, vorzugsweise zu mindestens 95 Gew.-% und insbesondere zu mindestens 97 Gew.-% aus den Monomeren N-tert-Octylacrylamid, Acrylsäure, tert.-Butylaminoethylmethacrylat, Methylmethacrylat und Hydroxypropylmethacrylat besteht.

Die zuvor beschriebenen amphoteren Copolymere werden beispielsweise unter der Bezeichnung Amphomer^{®} (INCI-Bezeichnung: Octylacrylamide / Acrylates / Butylaminoethyl Methacrylate Copolymer; CAS Nummer 70801-07-9) von der Firma National Starch vertrieben.

Ein vierter wesentlicher Bestandteil der kosmetischen Zusammensetzungen ist das Treibmittel. Als kosmetische und technisch besonders vorteilhaft hat es sich erwiesen, wenn der Gewichtsanteil des Treibmittels am Gesamtgewicht der Zusammensetzung 25 bis 50 Gew.-%, vorzugweise 30 bis 50 Gew.-% und insbesondere 35 bis 45 Gew.-% beträgt.

Bevorzugte Treibmittel (Treibgase) sind ausgewählt aus Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Bevorzugt ist der Einsatz von Propan, Propan/Butan-Gemischen oder Dimethylether, besonders bevorzugt der Einsatz von Dimethylether.

Die Zusammensetzung einiger bevorzugter kosmetischer Zusammensetzungen kann der folgenden Übersicht 1 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1a | Formel 2a | Formel 3a | Formel 4a | Formel 5a |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1b | Formel 2b | Formel 3b | Formel 4b | Formel 5b |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6a | Formel 7a | Formel 8a | Formel 9a | Formel 10a |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6b | Formel 7b | Formel 8b | Formel 9b | Formel 10b |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11a | Formel 12a | Formel 13a | Formel 14a | Formel 15a |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11b | Formel 12b | Formel 13b | Formel 14b | Formel 15b |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16a | Formel 17a | Formel 18a | Formel 19 | Formel 20a |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16b | Formel 17b | Formel 18b | Formel 19b | Formel 20b |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21a | Formel 22a | Formel 23a | Formel 24a | Formel 25a |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21b | Formel 22b | Formel 23b | Formel 24b | Formel 25b |
|---|---|---|---|---|---|
| Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26a | Formel 27a | Formel 28a | Formel 29a | Formel 30a |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26b | Formel 27b | Formel 28b | Formel 29b | Formel 30b |
|---|---|---|---|---|---|
| Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31a | Formel 32a | Formel 33a | Formel 34a | Formel 35a |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31b | Formel 32b | Formel 33b | Formel 34b | Formel 35b |
|---|---|---|---|---|---|
| Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36a | Formel 37a | Formel 38a | Formel 39a | Formel 40a |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36b | Formel 37b | Formel 38b | Formel 39b | Formel 40b |
|---|---|---|---|---|---|
| Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

Die erfindungsgemäßen kosmetischen Zusammensetzungen enthalten ferner
0 bis 10 Gew.-% Farbstoff(e)
0 bis 10 Gew.-% Pigment(e) enthält, mit der Maßgabe, dass
die Summe der Mengen der Inhaltsstoffe (e) und (f) 0,0001 bis 10 Gew.-%, bezogen auf das Gesamtgewicht der kosmetischen Zusammensetzung, beträgt.

In anderen Worten können die Zusammensetzungen O Gew.-% Farbstoffe enthalten (dann muß mindestens ein Pigment in einer Menge von mindestens 0,0001 Gew.-% vorhanden sein), der Farbstoffgehalt kann aber auch 10 Gew.-% betragen (dann kann kein Pigment mehr vorhanden sein. Unabhängig davon, ob
- nur ein Farbstoff,
- mehrere Farbstoffe,
- nur ein Pigment,
- mehrere Pigmente,
- ein Farbstoff und mehrere Pigmente,
- mehrere Farbstoffe und ein Pigment,
- mehrere Farbstoffe und mehrere Pigmente
eingesetzt werden, beträgt die Gesamtmenge aller Farbstoffe und Pigmente (auch als Gesamtmenge farbgebender Komponenten bezeichnet) in der kosmetischen Zusammensetzung 0,0001 bis 10 Gew.-%, bezogen auf das Gesamtgewicht der kosmetischen Zusammensetzung.

Es hat sich gezeigt, dass solche eingefärbten und/oder mit Farbstoffen bzw. Pigmenten versetzten kosmetischen Zusammensetzungen nicht nur das Haarvolumen erhöhen und eine Erhöhung der Fülle und des Gefühls "mehr Haare zu haben" erreichen, sondern dass die Färbung der Produkte gleichzeitig eine Abdeckung kahler Stellen ermöglicht werden kann, ohne dass es zu Abrieb auf die Hände oder Kleidung bzw. Kopfkissen kommt.

Bevorzugte kosmetische Zusammensetzungen sind dadurch gekennzeichnet, dass sie 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf das Gesamtgewicht der kosmetischen Zusammensetzung, Farbstoff(e) enthalten.

Farbstoffe sind Farbmittel, also farbgebende Substanzen, die im Gegensatz zu Pigmenten im Anwendungsmedium löslich sind (wie in Wasser, Ölen oder anderen Lösungsmitteln).

Je nach gewünschter Farbe, Farbintensität und Echtheitseigenschaften der resultierenden Färbung kann ein Farbstoff oder eine Mischung von Farbstoffen ausgewählt werden. Dabei sind alle auf dem Gebiet der Kosmetik üblichen und kommerziell erhältlichen Farbstoffe im Rahmen der vorliegenden Erfindung problemlos einsetzbar.

Ebenfalls bevorzugte erfindungsgemäße kosmetische Zusammensetzungen sind dadurch gekennzeichnet, dass 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% .-%, jeweils bezogen auf das Gesamtgewicht der kosmetischen Zusammensetzung, Pigment(e) enthalten.

Grundsätzlich eigenen sich alle Arten von wasserunlöslichen Pigmenten, beispielsweise natürliche anorganische Pigmente (auch als Mineralpigmente bezeichnet). Diese Pigmente beinhalten überwiegend Sulfide und Oxide. Beispiele für derartige Pigmente sind Ocker (Fe(OOH); Pigment Yellow 43), gebrannte Siena (Fe₂O₃; Pigment Red 102), Umbra (Fe₂O₃ x MnO₂; Pigment Brown 7:x), Zinnober (β-HgS, PR 106), Lapislazuli (Ultramarin, Na₆Al₆Si₆O₂₄ x Na₂Sn; Pigment Blue 29), Azurit (basisches Kupfercarbonat, Cu₃[OH/CO₃]₂; PB 30), Grünerde (FeO-haltiges Silicat; Pigment Green 23), Malachit (Cu₂[(OH)₂, CO₃]) und Kohlenschwarz (Kohlenstoff (Graphit), Pigment Black 9). In Bezug auf die Vermeidung von unerwünschten sichtbaren Rückständen oder Grauschleiern bzw. die wasserbeständige temporäre Färbung der Fasern hat sich jedoch der Einsatz von synthetischen anorganischen Pigmenten als vorteilhaft erwiesen. Synthetische anorganische Pigmente werden beispielsweise durch chemische und/oder physikalische Umwandlung (Aufschluss, Fällung, Glühen) hergestellt. Hierzu zählen insbesondere
- Weißpigmente (Titandioxid (TiO₂), Pigment White PW 6; Zinksulfid (ZnS), PW 7; Zinkoxid (ZnO), PW 4; Antimonweiß (Sb₂O₃), PW 11; Lithopone (ZnS/BaSO₄), PW 5; Bleiweiß (2PbCO₃ x Pb(OH)₂), PW 1),
- untergeordnet Weiß-Füllstoffe (Calciumcarbonat, PW 18; Talkum, PW 26 und Bariumsulfat, PW 21);
- Schwarzpigmente (Manganschwarz, Spinellschwarz sowie Pigmentruße (Graphit-Kohlenstoff);
- Glanzpigmente (Absorptionspigmente, Metallpigmente oder Metalleffektpigmente und Perlglanzpigmente) sowie
- anorganische Buntpigmente (Eisenoxid-Pigmente, Eisenblau-Pigmente, Ultramarin-Pigmente sowie die aufgrund ihrer toxikologischen Eigenschaften weniger geeigneten Bleichromat-Pigmente, Chromoxid-Pigmente, Cadmium-Pigmente und Bismutvanadat-Pigmente).

Bevorzugte synthetische anorganische Pigmente sind Metallpigmente oder Metalleffektpigmente aus pulverigen Metallen oder Metalllegierungen, wie Aluminiumbronzen (Metall: Al), Goldbronzen (Metall: Cu, Cu-Al- oder Cu-Zn-Legierung), Silberbronzen (Metall: Cu-Zn-Ni), feuergefärbte Bronzen (Metall: oxidiertes Cu-Zn) sowie Patentbronzen (Metall: Cu-Zn-(Ni) + Farbstoff).

Weitere bevorzugte synthetische anorganische Pigmente sind Perlglanzpigmente, welche aus mehreren Schichten mit unterschiedlicher Brechzahl bestehen. Beispiele für derartige Perlglanzpigmente sind Magnesiumstearat, Zinkstearat und Lithiumstearat oder Ethylenglycoldistearat bzw. Polyethylenterephthalat sowie Perlglanzpigmente, welche im Wesentlichen aus Glimmer, Titandioxid (Titandioxid-Glimmer), Bismutchloridoxid oder Guanin bestehen, und darüber hinaus mit farbigen Oxidschichten (z. B. Eisenoxiden oder Chromoxide) überzogen sein können. Perlglanzpigmente auf Glimmer-Basis und auf Glimmer/Metalloxid-Basis sind dabei erfindungsgemäß besonders bevorzugte Perlglanzpigmente. Glimmer gehören zu den Schicht-Silikaten. Die wichtigsten Vertreter dieser Silikate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäß bevorzugte Perlglanzpigmente erhalten. Diese Perlglanzpigmentarten weisen neben einem glitzernden optischen Effekt zusätzlich Farbeffekte auf. Des Weiteren können die erfindungsgemäß verwendbaren Perlglanzpigmente zusätzlich ein Farbpigment enthalten, welches sich nicht von einem Metalloxid ableitet.

Ganz besonders bevorzugte Perlglanzpigmente sind Pigmente, welche von der Firma Merck unter den Handelsnamen Colorona^{®} vermarktet werden, wobei die Pigmente Colorona^{®} red-brown (47-57 Gew.% Muscovit Mica (KH₂(AlSiO₄)₃), 43-50 Gew.% Fe₂O₃ (INCI: Iron Oxides CI 77491), <3 Gew.% TiO2 (INCi: Titanium Dioxide CI 77891), Colorona^{®} Blackstar Blue (39-47 Gew.% Muscovit Mica (KH2(AlSiO4)3), 53-61 Gew.% Fe3O4 (INCi: Iran Oxides CI 77499)), Colorona^{®} Siena Fine (35-45 Gew.% Muscovit Mica (KH2(AlSiO4)3), 55-65 Gew.% Fe2O3 (INCi: Iran Oxides CI 77491)), Colorona^{®} Aborigine Amber (50-62 Gew.% Muscovit Mica (KH2(AlSiO4)3), 36-44 Gew.% Fe3O4 (INCi: Iran Oxides CI 77499), 2-6 Gew.% TiO2 (INCi: Titanium Dioxide CI 77891)), Colorona^{®} Patagonian Purple (42-54 Gew.% Muscovit Mica (KH2(AlSiO4)3), 26-32 Gew.% Fe2O3 (INCi: Iran Oxides CI 77491), 18-22 Gew.% TiO2 (INCi: Titanium Dioxide CI 77891), 2-4 Gew.% Preussisch Blau (INCi: Ferric Ferrocyanide CI 77510)), Colorona^{®} Chameleon (40-50 Gew.% Muscovit Mica (KH2(AlSiO4)3), 50-60 Gew.% Fe2O3 (INCi: Iran Oxides CI 77491)) und Silk^{®} Mica ( >98 Gew.% Muscovit Mica (KH2(AlSiO4)3)) besonders bevorzugt sind.

Eine Gruppe besonders bevorzugter Pigmente bilden die farbgebenden synthetischen Eisenoxide. Besonders bevorzugte Vertreter dieser Substanzklasse sind Pigment Brown 6 (CI No 77491), Pigment Red 101 (CI No 77491), Pigment Yellow 42 (CI No 77492), Pigment Black 11 (CI No 77499) sowie Mischungen dieser Pigmente.

Ganz besonders bevorzugt einsetzbare Pigmente zeichnen sich durch eine gute Versprühbarkeit in den erfindungsgemäßen Zusammensetzungen, ein homogenes Sprühbild sowie eine äußerst geringe Neigung zum Verstopfen der Sprühdüsen ("clogging") aus. Hier sind erfindungsgemäß äußerst bevorzugte kosmetische Zusammensetzungen dadurch gekennzeichnet, dass sie mindestens ein Pigment aus der Gruppe CI12490, CI14700, CI14720, CI15510, CI15985, CI45380, CI47005, CI60730, CI61565, CI73360, CI74160, CI77007, CI77019, CI77288, CI77289, CI77491, CI77492 enthalten, wobei die Gesamtmenge an Pigment(en) aus dieser Gruppe 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-% beträgt.

Die erfindungsgemäßen Zusammensetzungen enthalten 0,1 bis 10 Gew.-% mindestens einer Stärkeverbindung. Diese Stärkeverbindung kann das einzige Polymer in den erfindungsgemäßen Mitteln sein, es ist aber bevorzugt, die Stärkeverbindung zusätzlich zu den weiter oben beschriebenen vorzugsweise filmbildenden Polymeren einzusetzen. Die Formulierung "als weiteren Bestandteil" bedeutet daher "zusätzlich zu" allen anderen vorstehend genannten zwingend erforderlichen Bestandteilen.

Bevorzugte kosmetische Zusammensetzungen sind dadurch gekennzeichnet, dass sie als weiteren Bestandteil 2,0 bis 6,0 Gew.-% und insbesondere 3,0 bis 5,0 Gew.- % mindestens einer Stärkeverbindung enthalten.

Stärke ist ein Reservekohlenhydrat, das von vielen Pflanzen in Form von üblicherweise 1 bis 200 µm großen Stärkekörnern (Granula) in verschiedenen Pflanzenteilen gespeichert wird, z.B. in Knollen oder Wurzeln, Getreidesamen, Früchten sowie im Mark. Stärke gehört zu der Familie der Homoglycane und ist ein Polykondensationsprodukt von D-Glucose. Erfindungsgemäß bevorzugte Stärken werden ausgewählt unter mindestens einem Polykondensationsprodukt von D-Glucose erhalten aus nativen und/oder physikalisch modifizierten Stärke von Kartoffeln, Mais, Reis, Erbsen, Eicheln, Kastanien, Gerste, Weizen, Bananen, Sago, Hirse, Sorghum, Hafer, Roggen, Bohnen, Batate, Maranta oder Maniok sowie deren Mischungen. In Bezug auf die Reinigungsleistung hat sich der Einsatz von nativer und/oder physikalisch modifizierter Reisstärke als besonders vorteilhaft erwiesen. Unter nativer Stärke wird eine Stärke verstanden, welche aus Stärke-haltigen Pflanzen isoliert wird und welche nach der Isolation und Aufreinigung weder physikalisch noch chemisch modifiziert wurde. Hingegen wird unter physikalisch modifizierter Stärke eine Stärke verstanden, welche nach der Isolierung mindestens einer physikalischen Modifikation unterworfen wurde. Unter physikalischer Modifikation ist hierbei die Modifikation unter Anwendung von Druck und/oder Hitze und/oder Licht zu verstehen. Eine Modifikation mittels chemischer und enzymatischer Reaktionen, beispielsweise die Hydrolyse der Stärke, fällt hierbei jedoch nicht unter den Begriff der physikalischen Modifikation. Eine bevorzugt eingesetzte physikalische Modifikation ist die Anwendung von Hitze, insbesondere das Kochen der nativen Stärke. Bevorzugte Ausführungsformen der vorliegenden Erfindung sind daher dadurch gekennzeichnet, dass die Zusammensetzungen mindestens eine Stärke, ausgewählt aus chemisch und/oder physikalisch modifizierten Reisstärken, insbesondere aus physikalisch modifizierter Reisstärke, enthalten. Durch den Einsatz von nativen und/oder physikalisch modifizierten Reisstärken in Kombination mit Farbstoffen und/oder Pigmenten wird eine besonders hohe Haftung der Farbstoffe und/oder Pigmente auf der Stärke erzielt, so dass neben der hervorragenden Reinigungsleistung auch eine lang anhaltende temporäre Haarfärbung ermöglicht wird.

Hierdurch ist es möglich, vorgefärbte und/oder vorpigmentierte Stärken einzusetzen, die sich mit den kosmetischen Zusammensetzungen herausragend versprühen lassen. Die erfindungsgemäßen kosmetischen Zusammensetzungen sind daher dadurch gekennzeichnet, dass sie den Inhaltsstoff (e) und/oder (f) in Form eines Stärke-Farbstoff-Compounds und/oder Stärke-Pigment- Compounds, vorzugsweise in Form von gefärbter und/oder pigmentierter Reisstärke, enthalten.

Es hat sich zusätzlich herausgestellt, dass sich die Zusammensetzung der Stärkepartikel selbst als für die kosmetische Wirkung relevant erwiesen hat. Daher weisen bevorzugt eingesetzte Partikel einen bestimmten Anteil der nativen und/oder physikalisch modifizierten Stärke, insbesondere Reisstärke, auf. Es ist somit bevorzugt, wenn das Stärke-Farbstoff-Compound mindestens eine Stärke, insbesondere eine physikalisch modifizierte Reisstärke, in einer Gesamtmenge von 70 bis 96 Gew.-%, insbesondere von 80 bis 94 Gew.-%, jeweils bezogen auf das Gesamtgewicht des Partikels (Compounds), enthält. Der Einsatz von Partikeln, welche einen hohen Gewichtsanteil an physikalisch modifizierter Reisstärke enthalten, führt zu besonders homogenen Sprühbildern und bevorzugte Haftung der Farbe auf den keratinischen Fasern.

Zusätzlich zu solchen Compounds aus Stärke und farbgebenden Substanzen oder an ihrer Stelle können naturgemäß auch die Fasern der kosmetischen Zusammensetzungen vorgefärbt und/oder vorpigmentiert werden. Hier sind kosmetische Zusammensetzungen besonders bevorzugt, die die Inhaltsstoffe (a) und (e) und/oder (a) und (f) in Form eines Faser-Farbstoff-Compounds oder Faser-Pigment-Compounds, vorzugsweise in Form von gefärbten und/oder pigmentierten Fasern, enthalten.

Bevorzugte kosmetische Zusammensetzungen enthalten als weiteren Bestandteil mindestens ein Esteröl. Zur Gruppe der Esteröle zählen dabei insbesondere Ester von C₆ - C₃₀ - Fettsäuren mit C₂ - C₃₀ - Fettalkoholen, vorzugsweise Monoester der Fettsäuren mit Alkoholen mit 2 bis 24 C-Atomen. Beispielhafte Esteröle sind das Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN) und Ölsäuredecylester (Cetiol^{®} V). Besonders bevorzugt ist der Einsatz von Isopropylmyristat.

Der Gewichtsanteil des Esteröls am Gesamtgewicht der kosmetischen Zubereitung beträgt vorzugsweise 0,01 bis 1,0 Gew.-%, bevorzugt 0,02 bis 0,6 Gew.-% und insbesondere 0,03 bis 0,2 Gew.-%.

Die eingangs als erster wesentlicher Bestandteil der kosmetischen Zusammensetzungen beschriebenen Fasern können in unterschiedlicher Weise modifiziert werden. Derartige Modifikationen betreffen beispielsweise die weiter oben beschriebene Länge der Fasern oder deren Längen zu Durchmesser Verhältnis.

Eine weitere Möglichkeit, die technischen und kosmetischen Eigenschaften der Zusammensetzungen zu beeinflussen liegt in der Modifikation der Oberflächeneigenschaften der eingesetzten Fasern.

In einer ersten bevorzugten Ausführungsform werden hydrophobierte Fasern eingesetzt. Die Hydrophobierung (Imprägnierung) bewirkt eine wasserabstoßende Ausrüstung der Faser. Als Hydrophobierungsmittel eignen sich beispielsweise Paraffine oder Wachse aber auch filmbildende Silikone.

In Abhängigkeit vom Verwendungszweck der kosmetischen Zusammensetzung kann es von Vorteil sein, wenn die Fasern eine gewisse Ölabsorptionskapazität aufweisen. Diese Ölabsorptionskapazität kann mittels der zuvor beschriebenen Hydrophobierung der Fasern beeinflusst werden. Bevorzugt ist der Einsatz hydrophobierter oder nicht hydrophobierter Fasern mit einer Ölabsorptionskapazität oberhalb 0,2 g Jojobaöl pro Gramm Fasern. Besonders bevorzugt ist der Einsatz von Fasern mit einer Ölabsorptionskapazität (20°C) von 0,2 bis 0,8 g Jojobaöl/g, vorzugsweise von 0,3 bis 0,7 g Jojobaöl/g, besonders bevorzugt von 0,4 bis 0,6 g Jojobaöl/g.

Beispielsweise um eine gleichmäßige Suspension der Fasern in der kosmetischen Zusammensetzung zu ermöglichen oder die kosmetische Wirkung der Zusammensetzung zu steigern, können die Zusammensetzungen als weiteren optionalen Bestandteil Tensid umfassen. Bevorzugt ist der Einsatz nichtionischer oder kationischer Tenside.

Bevorzugte kationische Tensind sind ausgewählt aus der Gruppe der quartären Ammoniumverbindungen. Bevorzugte quartäre Ammoniumverbindungen sind wiederum Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Ganz besonders bevorzugt ist der Einsatz von Cetyltrimethylammoniumchlorid.

Der Gewichtsanteil des Tensids am Gesamtgewicht der kosmetischen Zusammensetzung beträgt vorzugsweise 0,05 bis 2,0 Gew.-%, bevorzugt 0,1 bis 1,5 Gew.-% und insbesondere 0,2 bis 1,0 Gew.-%.

Die Zusammensetzung einiger weiterer bevorzugter kosmetischer Zusammensetzungen kann der folgenden Übersicht 2 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1a | Formel 2a | Formel 3a | Formel 4a | Formel 5a |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1b | Formel 2b | Formel 3b | Formel 4b | Formel 5b |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6a | Formel 7a | Formel 8a | Formel 9a | Formel 10a |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6b | Formel 7b | Formel 8b | Formel 9b | Formel 10b |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11a | Formel 12a | Formel 13a | Formel 14a | Formel 15a |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11b | Formel 12b | Formel 13b | Formel 14b | Formel 15b |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16a | Formel 17a | Formel 18a | Formel 19 | Formel 20a |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16b | Formel 17b | Formel 18b | Formel 19b | Formel 20b |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21a | Formel 22a | Formel 23a | Formel 24a | Formel 25a |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21b | Formel 22b | Formel 23b | Formel 24b | Formel 25b |
|---|---|---|---|---|---|
| hydrophobierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26a | Formel 27a | Formel 28a | Formel 29a | Formel 30a |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26b | Formel 27b | Formel 28b | Formel 29b | Formel 30b |
|---|---|---|---|---|---|
| hydrophobierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31a | Formel 32a | Formel 33a | Formel 34a | Formel 35a |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31b | Formel 32b | Formel 33b | Formel 34b | Formel 35b |
|---|---|---|---|---|---|
| hydrophobierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36a | Formel 37a | Formel 38a | Formel 39a | Formel 40a |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36b | Formel 37b | Formel 38b | Formel 39b | Formel 40b |
|---|---|---|---|---|---|
| hydrophobierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |

| Ethanol | 20 bis 50 | 20 bis 50 | 20 bis 45 | 25 bis 40 | 25 bis 40 |
|---|---|---|---|---|---|
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

In einer alternativen Ausführungsform werden in den kosmetischen Mitteln hydrophilierte Fasern eingesetzt. Entsprechende Fasern verfügen gegenüber den nicht hydrophilierten Fasern über ein erhöhtes Wasseraufnahmevermögen.

Die Wasseradsorptionskapazität (20°C)bevorzugter Fasern, insbesondere bevorzugter hydrophilierter Fasern liegt vorzugsweise oberhalb 5,0 Gew.-%, vorzugsweise oberhalb 7,0 Gew.-% und insbesondere oberhalb 9,0 Gew.-% des Eigengewichts der Fasern.

Die Hydrophophilierung der Fasern verbessert u.a. deren Wasser-Aufnahmevermögen. Entsprechende Fasern werden daher insbesondere in auffrischenden kosmetischen Zusammensetzungen eingesetzt.

Die Zusammensetzung einiger weiterer bevorzugter kosmetischer Zusammensetzungen kann der folgenden Übersicht 3 entnommen werden (Angaben in Gew.-% bezogen auf das Gesamtgewicht der kosmetischen Zusammensetzung).

| | Formel 1 | Formel 2 | Formel 3 | Formel 4 | Formel 5 |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1a | Formel 2a | Formel 3a | Formel 4a | Formel 5a |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 1b | Formel 2b | Formel 3b | Formel 4b | Formel 5b |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6 | Formel 7 | Formel 8 | Formel 9 | Formel 10 |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6a | Formel 7a | Formel 8a | Formel 9a | Formel 10a |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 6b | Formel 7b | Formel 8b | Formel 9b | Formel 10b |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11 | Formel 12 | Formel 13 | Formel 14 | Formel 15 |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11a | Formel 12a | Formel 13a | Formel 14a | Formel 15a |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 11b | Formel 12b | Formel 13b | Formel 14b | Formel 15b |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16 | Formel 17 | Formel 18 | Formel 19 | Formel 20 |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16a | Formel 17a | Formel 18a | Formel 19 | Formel 20a |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 16b | Formel 17b | Formel 18b | Formel 19b | Formel 20b |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Treibmittel | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21 | Formel 22 | Formel 23 | Formel 24 | Formel 25 |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21a | Formel 22a | Formel 23a | Formel 24a | Formel 25a |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 21b | Formel 22b | Formel 23b | Formel 24b | Formel 25b |
|---|---|---|---|---|---|
| hydrophilierte Faser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26 | Formel 27 | Formel 28 | Formel 29 | Formel 30 |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26a | Formel 27a | Formel 28a | Formel 29a | Formel 30a |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 26b | Formel 27b | Formel 28b | Formel 29b | Formel 30b |
|---|---|---|---|---|---|
| hydrophilierte Cellulosefaser | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31 | Formel 32 | Formel 33 | Formel 34 | Formel 35 |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31a | Formel 32a | Formel 33a | Formel 34a | Formel 35a |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 31b | Formel 32b | Formel 33b | Formel 34b | Formel 35b |
|---|---|---|---|---|---|
| hydrophilierte Faser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36 | Formel 37 | Formel 38 | Formel 39 | Formel 40 |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36a | Formel 37a | Formel 38a | Formel 39a | Formel 40a |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| Polyvinylpyrrolidon, Vinylpyrrolidon/Vinylacetat-Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

| | Formel 36b | Formel 37b | Formel 38b | Formel 39b | Formel 40b |
|---|---|---|---|---|---|
| hydrophilierte Cellulosefaser mit einer Faserlänge zwischen 1,0 und 200 µm | 0,1 bis 5,0 | 0,1 bis 2,0 | 0,1 bis 2,0 | 0,1 bis 1,0 | 0,1 bis 0,5 |
| Ethanol | 20 bis 50 | 20 bis 50 | 20 bis 45 | 25 bis 40 | 25 bis 40 |
| N-tert-Octylacrylamid, Acrylsäure, tert.-Butylaminoethylmethacrylat Copolymer | 0,5 bis 8,0 | 0,5 bis 8,0 | 1,0 bis 6,0 | 1,0 bis 6,0 | 2,0 bis 4,0 |
| Dimethylether | 30 bis 70 | 35 bis 65 | 35 bis 65 | 40 bis 60 | 40 bis 60 |
| Optionale Additive | ad 100 | ad 100 | ad 100 | ad 100 | ad 100 |

In einer weiteren alternativen Ausführungsform enthalten bevorzugte kosmetische Zusammensetzungen mindestens einen Duftstoff.

Als optionale weitere geeignete Wirk- oder Hilfsstoffe, welche in den kosmetischen Zusammensetzungen enthalten sein können, sind insbesondere zusätzliche Pflegestoffe zu nennen.

Als Pflegestoff kann die Zusammensetzung beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann die kosmetische Zusammensetzung weiterhin mindestens ein Vitamin, ein Provitamin, eine Vitaminvorstufe und/oder eines derer Derivate enthalten. Dabei sind solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die kosmetischen zusammensetzungen weiterhin mindestens einen Pflanzenextrakt, aber auch Mono- bzw. Oligosaccharide und/oder Lipide enthalten.

Zur Abgabe und Applikation der kosmetischen Zusammensetzungen eignen sich insbesondere Aerosolabgabebehälter. Ein weiterer Anmeldungsgegenstand sind dem entsprechend kosmetische Mittel, umfassend
a) eine erfindungsgemäßen kosmetische Zusammensetzung
b) einen Aerosolabgabebehälter.

Unter der Bezeichnung Aerosolabgabebehälter sind Druckbehälter zu verstehen, in deren Inneren ein höherer Gasdruck herrscht als außerhalb des Behälters und aus dem sich ein Gasstrom über ein Ventil entnehmen lässt. Bei den Aerosolabgabebehälter, handelt es sich mit anderen Worten um Druckbehälter, mit deren Hilfe ein Produkt (z.B. eine kosmetische Zusammensetzung) durch den inneren Gasdruck des Behälters über ein Ventil abgegeben werden kann.

Die kosmetischen Zusammensetzungen lassen sich in üblicher Art und Weise herstellen. In der Regel werden alle Bestandteile der kosmetischen Zusammensetzung mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge des Treibmittels eingefüllt.

Als druckfeste Behälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der im Druckbehälter konfektionierten Zusammensetzung. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Als Aerosolabgabebehälter kann auch ein Mehrkammerspender eingesetzt werden. Der Mehrkammerspender kann auch so ausgestaltet sein, dass eine Kammer das komprimierte Treibmittel und eine andere Kammer mit den restlichen Bestandteilen der erfindungsgemäßen Zusammensetzung befüllt ist. Ein derartiger Mehrkammerspender ist beispielsweise eine so genannte Bag-in-Can-Verpackung.

Die Sprührate der kosmetischen Mittel beträgt bevorzugt 6,5 bis 10,0 g/10 s.

Wie weiter oben ausgeführt, eignen sich die die erfindungsgemäßen Zusammensetzungen insbesondere zur Reinigung, Auffrischung und Desodorierung keratinischer Fasern. Entsprechende Verwendungen der kosmetischen Zusammensetzungen sind weitere Gegenstände dieser Anmeldung.

Ein letzter Gegenstand dieser Anmeldung ist ein Verfahren zur temporären Verformung von keratinischen Fasern, insbesondere menschlichen Haaren, bei welchem eine erfindungsgemäße kosmetische Zusammensetzung auf keratinische Fasern appliziert wird. Für die Bestandteile dieser Zusammensetzung, deren Gewichtsanteile und bevorzugte Ausführungsformen gilt mutatis mutandis das zuvor Gesagte.

## Patentansprüche

1. Eine kosmetische Zusammensetzung zur kosmetischen Behandlung keratinischer Fasern, umfassend:
(a) Fasern, wobei die Fasern ausgewählt sind aus Baumwollfasern, Kapokfasern, Akonfasern, Pappelflaumfasern, Bambusfasern, Fasernessel, Hanffaser, Jutefaser, Flachsfaser, Sisalfaser, Kokosfaser und Fasern von Wolle, Alpaka, Kamelhaar, Amgora, Kaschmir, Mohair, Yakhaar, Ziegenhaar, Rinderhaar und Rosshaar, bevorzugt aus Cellulosefasern.
(b) Ethanol, wobei der Gewichtsanteil des Ethanols am Gesamtgewicht der kosmetischen Zusammensetzung 20 bis 50 Gew.-%, vorzugsweise 20 bis 45 Gew.-% und insbesondere 25 bis 40 Gew.-% beträgt,
(c) mindestens ein Polymer,
(d) Treibmittel,
(e) 0 bis 10 Gew.-% Farbstoff(e),
(f) 0 bis 10 Gew.-% Pigment(e),
(g) 1 ,0 bis 10 Gew.-%, mindestens einer Stärkeverbindung
enthält, mit der Maßgabe, dass
die Summe der Mengen der Inhaltsstoffe (e) und (f) 0,0001 bis 10 Gew.-%, bezogen auf das Gesamtgewicht der kosmetischen Zusammensetzung, beträgt und
wobei der Inhaltsstoff (e) und/oder (f) in Form eines Stärke-Farbstoff-Compounds und/oder eines Stärke-Pigment-Compounds, vorzugsweise in Form von gefärbter und/oder pigmentierter Reisstärke, enthalten ist.

2. Kosmetische Zusammensetzung nach Anspruch 1, wobei der Gewichtsanteil der Fasern am Gesamtgewicht der kosmetischen Zusammensetzung 0, 1 bis 5,0, bevorzugt 0,1 bis 2,0 Gew.-%, besonders bevorzugt 0, 1 bis 1 ,0 Gew.-% und insbesondere 0, 1 bis 0,5 Gew.-% beträgt.

3. Kosmetische Zusammensetzung nach einem der vorherigen Ansprüche, wobei die Fasern ausgewählt sind aus der Gruppe der pflanzlichen Fasern, insbesondere der Cellulosefasern, vorzugsweise der Baumwollfasern, Flachsfasern und Kapopkfasern.

4. Kosmetische Zusammensetzung nach einem der vorherigen Ansprüche, wobei die Fasern eine Länge von 1 ,0 bis 200 µ 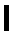 n, vorzugsweise von 2,0 bis 150 µm und insbesondere von 5,0 bis 100 µm aufweisen.

5. Kosmetische Zusammensetzung nach einem der vorherigen Ansprüche, wobei der Gewichtsanteil des mindestens einen Polymers am Gesamtgewicht der kosmetischen Zusammensetzung 0,5 bis 8,0 Gew.-%, vorzugsweise 1 ,0 bis 6,0 Gew.-% und insbesondere 2,0 bis 4,0 Gew.-% beträgt.

6. Kosmetische Zusammensetzung nach einem der vorherigen Ansprüche, wobei der Gewichtsanteil des Treibmittels am Gesamtgewicht des kosmetischen Zusammensetzung 30 bis 70 Gew.-%, vorzugweise 35 bis 65 Gew.-% und insbesondere 40 bis 60 Gew.-% beträgt.

7. Kosmetische Zusammensetzung nach einem der Ansprüche 2 bis 6, wobei sie - bezogen auf ihr Gesamtgewicht - 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.- % Farbstoff(e) enthält.

8. Kosmetische Zusammensetzung nach einem der Ansprüche 2 bis 7, wobei sie - bezogen auf ihr Gesamtgewicht - 0,0001 bis 10 Gew.-%, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.- % Pigment(e) enthält.

9. Kosmetische Zusammensetzung nach einem der vorherigen Ansprüche 2 bis 8, wobei sie mindestens ein Pigment aus der Gruppe CI 12490, CI 14700, CI 14720, CI15510, CI15985, CI45380, CI47005, CI60730, CI61565, CI73360, CI74160, CI77007, CI77019, CI77288, CI77289, CI77491 , CI77492 enthält, wobei die Gesamtmenge an Pigment(en) aus dieser Gruppe 0,0001 bis 10 Gew.- %, vorzugsweise 0,01 bis 9,5 Gew-%, weiter bevorzugt 0,05 bis 9 Gew.-%, noch weiter bevorzugt 0,1 bis 8,5 Gew.-% und insbesondere 0,25 bis 8 Gew.-%, jeweils bezogen auf das Gesamtgewicht der kosmetischen Zusammensetzung, beträgt.

10. Kosmetische Zusammensetzung nach einem der vorherigen Ansprüche, wobei sie als weiteren Bestandteil 2,0 bis 6,0 Gew.-% und insbesondere 3,0 bis 5,0 Gew.- %, jeweils bezogen auf das Gesamtgewicht der kosmetischen Zusammensetzung, mindestens einer Stärkeverbindung enthält.

11. Kosmetische Zusammensetzung nach einem der vorherigen Ansprüche 2 bis 10, wobei sie die Inhaltsstoffe (a) und (e) und/oder (a) und (f) in Form eines Faser-Farbstoff-Compounds oder eines Faser-Pigment-Compounds, vorzugsweise in Form von gefärbten und/oder pigmentierten Fasern, enthält.

12. Kosmetisches Mittel, umfassend
a) eine kosmetische Zusammensetzung nach einem der Ansprüche 1 bis 11
b) einen Aerosolabgabebehälter.

## Claims

1. A cosmetic composition for the cosmetic treatment of keratinous fibres comprising:
(a) fibres, wherein the fibres are selected from cotton fibres, kapok fibres, acon fibres, poplar fluff fibres, bamboo fibres, fibre nettle, hemp fibre, jute fibre, flax fibre, sisal fibre, coconut fibre and fibres of wool, alpaca, camel hair, amgora, cashmere, mohair, yak hair, goat hair, cow hair and horsehair, preferably from cellulose fibres.
(b) ethanol, wherein the proportion by weight of ethanol in the total weight of the cosmetic composition being 20 to 50% by weight, preferably 20 to 45% by weight and in particular is 25 to 40 % by weight,
(c) at least one polymer,
(d) propellant,
(e) 0 to 10 % by weight of colourant(s),
(f) 0 to 10 % by weight of pigment(s),
(g) 1.0 to 10 % by weight of at least one starch compound,
with the proviso that
the sum of the amounts of ingredients (e) and (f) is 0.0001 to 10% by weight, based on the total weight of the cosmetic composition, and
wherein the ingredient (e) and/or (f) is present in the form of a starch-colourant compound and/or a starch-pigmentcompound, preferably in the form of coloured and/or pigmented rice starch.

2. Cosmetic composition according to claim 1, wherein the proportion by weight of the fibres in the total weight of the cosmetic composition is 0,1 to 5,0, preferably 0,1 to 2,0 % by weight, more preferably 0,1 to 1,0 % by weight and even more preferably 0,1 to 0,5 % by weight.

3. Cosmetic composition according to any one of the preceding claims, wherein the fibres are selected from the group of vegetable fibres, in particular cellulose fibres, preferably cotton fibres, flax fibres and kapopk fibres.

4. Cosmetic composition according to any one of the preceding claims, wherein the fibres have a length of from 1,0 to 200 µm, preferably from 2,0 to 150 µm and more preferably from 5,0 to 100 µm.

5. Cosmetic composition according to one of the preceding claims, wherein the proportion by weight of the at least one polymer in the total weight of the cosmetic composition is 0,5 to 8,0 % by weight, preferably 1,0 to 6.0 % by weight and more preferably 2,0 to 4,0 % by weight.

6. Cosmetic composition according to any one of the preceding claims, wherein the proportion by weight of the propellant in the total weight of the cosmetic composition is 30 to 70 % by weight, preferably 35 to 65 % by weight and particularly preferably 40 to 60 % by weight.

7. Cosmetic composition according to any one of claims 2 to 6, wherein it contains - based on its total weight - 0,0001 to 10 % by weight, preferably 0,01 to 9,5 % by weight, more preferably 0,05 to 9 % by weight, even more preferably 0,1 to 8,5 % by weight and in particular 0,25 to 8 % by weight contains colourant(s).

8. Cosmetic composition according to any one of claims 2 to 7, wherein it contains - based on its total weight - 0,0001 to 10% by weight, preferably 0,01 to 9.5% by weight, more preferably 0.05 to 9 wt.%, even more preferably 0.1 to 8.5 wt.% and in particular 0.25 to 8 wt.%. % pigment(s).

9. Cosmetic composition according to any one of the preceding claims 2 to 8, wherein it contains at least one pigment from the group CI 12490, CI 14700, CI 14720, CI15510, CI15985, CI45380, CI47005, CI60730, CI61565, CI73360, CI74160, CI77007, CI77019, CI77288, CI77289, CI77491, CI77492, wherein the total amount of pigment(s) from this group is 0.0001 to 10 % by weight, preferably 0.01 to 9.5 % by weight, more preferably 0.05 to 9 % by weight, still more preferably 0.1 to 8.5 % by weight and in particular 0.25 to 8 % by weight, in each case based on the total weight of the cosmetic composition.

10. Cosmetic composition according to one of the preceding claims, wherein it contains as a further constituent 2,0 to 6,0 % by weight and preferably 3,0 to 5,0 % by weight, in each case based on the total weight of the cosmetic composition, of at least one starch compound.

11. Cosmetic composition according to any one of the preceding claims 2 to 10, wherein it contains the ingredients (a) and (e) and/or (a) and (f) in the form of a fibre-colourant compound or a fibre-pigment compound, preferably in the form of coloured and/or pigmented fibres.

12. Cosmetic product, comprising
a) a cosmetic composition according to any one of claims 1 to 11
b) an aerosol dispenser.

## Revendications

1. Une composition cosmétique pour le traitement cosmétique des fibres kératiniques, comprenant :
(a) Fibres, lesdites fibres étant choisies parmi les fibres de coton, les fibres de kapok, les fibres d'akon, les fibres de duvet de peuplier, les fibres de bambou, les fibres d'ortie, les fibres de chanvre, les fibres de jute, les fibres de lin, les fibres de sisal, les fibres de coco et les fibres de laine, d'alpaga, de poil de chameau, d'amgora, de cachemire, de mohair, de poil de yak, de poil de chèvre, de poil de bovin et de crin de cheval, de préférence les fibres de cellulose.
(b) de l'éthanol, le pourcentage en poids de l'éthanol par rapport au poids total de la composition cosmétique étant de 20 à 50 % en poids, de préférence de 20 à 45 % en poids et, en particulier
25 à 40 % en poids,
(c) au moins un polymère,
(d) agent gonflant,
(e) 0 à 10 % en poids de colorant(s),
(f) 0 à 10 % en poids de pigment(s),
(g) 1 ,0 à 10 % en poids d'au moins un composé d'amidon,
étant entendu que
la somme des quantités des ingrédients (e) et (f) est de 0,0001 à 10 % en poids, par rapport au poids total de la composition cosmétique, et
l'ingrédient (e) et/ou (f) étant contenu sous la forme d'un compound d'amidon et de colorant et/ou d'un compound d'amidon et de pigment, de préférence sous la forme d'amidon de riz coloré et/ou pigmenté.

2. Composition cosmétique selon la revendication 1, dans laquelle la proportion en poids des fibres par rapport au poids total de la composition cosmétique est de 0,1 à 5,0, de préférence de 0,1 à 2,0 % en poids, de préférence 0,1 à 1,0 % en poids et en particulier 0,1 à 0,5 % en poids.

3. Composition cosmétique selon l'une quelconque des revendications précédentes, dans laquelle les fibres sont choisies dans le groupe constitué par les fibres végétales, notamment les fibres de cellulose, de préférence les fibres de coton, les fibres de lin et les fibres de kapopk.

4. Composition cosmétique selon l'une quelconque des revendications précédentes, dans laquelle les fibres ont une longueur comprise entre 1 ,0 et 200 µ 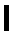 η , de préférence entre 2,0 et 150 µ 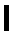 η et notamment entre 5,0 et 100 µ 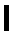 η.

5. Composition cosmétique selon l'une quelconque des revendications précédentes, dans laquelle le pourcentage en poids dudit au moins un polymère par rapport au poids total de la composition cosmétique est compris entre 0,5 et 8,0 % en poids, de préférence entre 1,0 et 6,0 % en poids, et plus particulièrement 2,0 à 4,0 % en poids.

6. Composition cosmétique selon l'une quelconque des revendications précédentes, dans laquelle la proportion en poids de l'agent propulseur par rapport au poids total de la composition cosmétique est de 30 % à 70 % en poids, de préférence de 35 à 65 % en poids et en particulier de 40 à 60 % en poids.

7. Composition cosmétique selon l'une quelconque des revendications 2 à 6, dans laquelle elle représente - par rapport à son poids total - de 0,0001 à 10 % en poids, de préférence de 0,01 à 9,5 % en poids, de manière encore plus préférée 0,05 à 9 % en poids, de préférence encore 0,1 à 8,5 % en poids et en particulier 0,25 à 8 % en poids % de colorant(s).

8. Composition cosmétique selon l'une quelconque des revendications 2 à 7, dans laquelle elle représente - par rapport à son poids total - de 0,0001 à 10 % en poids, de préférence de 0,01 à 9,5 % en poids, de manière encore plus préférée 0,05 à 9 % en poids, de préférence encore 0,1 à 8,5 % en poids et en particulier 0,25 à 8 % en poids % de pigment(s).

9. Composition cosmétique selon l'une quelconque des revendications précédentes 2 à 8, dans laquelle elle comprend au moins un pigment choisi dans le groupe constitué par CI 12490, CI 14700, CI 14720, CI15510, CI15985, CI45380, CI47005, CI60730, CI61565, CI73360, CI74160, CI77007, CI77019, CI77288, CI77289, CI77491, CI77492, la quantité totale de pigment(s) de ce groupe étant de 0,0001 à 10 % en poids, de préférence de 0,01 à 9,5 % en poids, plus préférentiellement de 0,05 à 9 % en poids, encore plus préférentiellement de 0,1 à 8,5 % en poids et en particulier de 0,25 à 8 % en poids, dans chaque cas par rapport au poids total de la composition cosmétique.

10. Composition cosmétique selon l'une quelconque des revendications précédentes, dans laquelle elle contient comme autre ingrédient de 2,0 à 6,0 % en poids, et en particulier de 3,0 à 5,0 % en poids, dans chaque cas par rapport au poids total de la composition cosmétique, d'au moins un composé d'amidon.

11. Composition cosmétique selon l'une quelconque des revendications précédentes 2 à 10, dans laquelle elle comprend les ingrédients (a) et (e) et/ou (a) et (f) sous forme d'un compound fibre-colorant ou d'un compound fibre-pigment, de préférence sous forme de fibres colorées et/ou pigmentées.

12. Produit cosmétique comprenant
a) une composition cosmétique selon l'une quelconque des revendications 1 à 11
b) un distributeur d'aérosols.
